# EUROPEAN PATENT SPECIFICATION

(11) **EP 3 969 090 B1**
(45) Date of publication and mention of the grant of the patent: **22.07.2026**
(21) Application number: 20805158.1
(22) Date of filing: 15.05.2020
(51) Int. Cl.: A61M 21/02, A61M 21/00, A61M 11/02, A61M 11/00, A61B 5/00, A61B 5/08, A61M 11/04, A61M 15/00, A61M 15/08, A61M 16/00

(54) **DEVICE AND METHOD TO SELECTIVELY PROVIDE AN ODOR STIMULATION**
VORRICHTUNG UND VERFAHREN ZUR SELEKTIVEN BEREITSTELLUNG EINER GERUCHSSTIMULIERUNG
DISPOSITIF ET PROCÉDÉ POUR FOURNIR SÉLECTIVEMENT UNE STIMULATION PAR ODEURS

(30) Priority: 15.05.2019 US 201962847979 P
(43) Date of publication of application: 23.03.2022
(73) Proprietor: Appscent Medical Ltd., 4366517 RaAnana (IL)
(72) Inventor: AZOULAY, Yosef, 4938978 Petah Tikva (IL); PORAT, Amos, 4335013 RaAnana (IL)
(74) Representative: Schwarz & Partner Patentanwälte GmbH
(86) International application number: PCT/IL2020/050534
(87) International publication number: WO 2020/230143

(56) References cited:
- WO-A1-2017/134166
- JP-A- 2007 289 298
- JP-A- 2016 518 165
- US-A1- 2012 272 958
- US-A1- 2012 272 958
- US-A1- 2013 190 556
- US-B1- 6 666 830
- US-B2- 7 528 102
- US-B2- 8 469 293
- A EIBENSTEIN, FIORETTI A B, LENA C, ROSATI N, OTTAVIANO I, FUSETTI M: "Olfactory screening test: experience in 102 Italian subjects", ACTA OTORHINOLARYNGOLOGICA ITALICA, vol. 25, no. 1, 25 February 2005 (2005-02-25), pages 18 - 22, XP055759711

## Description

### RELATED APPLICATION

This application claims the benefit of priority of U.S. Provisional Patent Application No. 62/847,979 filed on May 15, 2019.

### FIELD AND BACKGROUND OF THE INVENTION

The present invention, in some embodiments thereof, relates to odor stimulation for detecting and treating a respiratory conditions and, more particularly, but not exclusively, to treating apnea based on selective dispensing of odors.

Sleep apnea is a common but serious sleep disorder where breathing is briefly interrupted during sleep. The most common type of sleep apnea is obstructive sleep apnea. Obstructive sleep apnea occurs when the soft tissue in the back of the throat relaxes during sleep and blocks the airway. The blockage leads to pauses in breathing and loud snoring.

Continuous Positive Airflow Pressure (CPAP) is a known treatment for moderate to severe obstructive sleep apnea. The CPAP device is a mask-like machine that covers your nose and mouth, providing a stream of air that keeps your breathing passages open while you sleep. Many users find CPAP devices to be bulky, loud and generally uncomfortable to use.

U.S. Patent Application Publication No. 2012-0272958 entitled "Device and method for controlling respiration during sleep," describes a device for reducing a probability of snoring during sleep and/or an apnea event. Respiration is controlled by repeated dispersion of odors. It is disclosed that the device includes an odor disperser adapted to disperse an odor, a detector adapted to detect a physiological characteristic of a user and a controller. The controller is described as being configured to reduce the probability of snoring based on instructing the odor dispenser to disperse an odor responsive to detections by the detector. The odor dispensing affects one or more upcoming breaths of the user to increase the inhalation volume without inducing arousal. It is described that the device can be worn as a nose clip or that it may be integrated into a bed pillow.

U.S. Patent No. 6,666,830 entitled "System and method for detecting the onset of an obstructive sleep apnea event," describes a system and method for detecting the onset of an obstructive sleep apnea event before the obstructive sleep apnea event fully develops and before the cessation of breathing occurs. The system comprises one or more microphones capable of detecting breathing sounds within an airway of a person. The microphones generate signals representative of the breathing sounds and send the signals to a controller. The controller identifies at least one signal pattern that is associated with a breathing pattern of the person that occurs at the onset of an obstructive sleep apnea event. The controller may also identify at least one signal pattern that is associated with a partially occluded breathing pattern of the person. The controller identifies the signal patterns by using digital signal processing techniques to analyze the signals representative of breathing sounds. The method involves detecting breathing sounds within an airway of a person, generating signals representative of the breathing sounds, and identifying at least one signal pattern that is associated with a breathing pattern of the person that occurs at the onset of an obstructive sleep apnea event.

U.S. Patent No. 8,679,030 entitled "Monitoring a condition of a subject," describes a method of predicting an onset of apnea. Motion of a subject, including at least breathing-related motion, is sensed, and a signal corresponding to the sensed motion is generated. A breathing-related signal is extracted from the sensed motion signal, and the onset of apnea is predicted at least partially in response to analyzing the breathing-related signal. Other applications are also described. A user interface that is adapted to notify the subject and/or a healthcare worker of the predicted or occurring event is disclosed. Prediction of an approaching clinical event facilitates early preventive treatment, which generally reduces the required dosage of medication, and/or lowers mortality and morbidity. Further relevant prior art documents are: JP 2007 289298 A, WO 2017/134166 A1 and JP 2016 518165 A

### SUMMARY OF THE INVENTION

The present invention provides a device to selectively provide an odor stimulation according to claim 1.

A common method to dispense an odor in a room is based on atomizing a liquid in the air. The atomized liquid is typically configured to remain suspended in the air over an extended duration. For example, in an application such as an air freshener it is desired to maintain the scent in the air for as long as possible. However, when dispensing odors to provide a stimulation to detect and/or treat a respiratory condition quick dissipation of the odor after it is dispensed may be preferred. Quick dissipation may provide a burst stimulation, a spike scent and/or spike stimulation that avoids adaption of the user to the odor stimulus and/or may avoid saturation of the room with the odor. The adaption and/or saturation may lead to an undesired need for increasing the dose of odor material over time to obtain a same reaction to the stimulation. When progressively increasing the dose, control of the odor stimulation may be compromised, cost of operation may be increased and other occupants in the room may be disturbed by the odor accumulation in the room.

According to some example embodiments, effectiveness and controllability of odor stimulation to detect and/or treat a respiratory condition is improved based on affecting quick dissipation of the odor that is suspended in the air. The quick dissipation may avoid adaption of the user and saturation of the room so that a desired reaction in respiration may be achieved with smaller doses of odorized material and in a more controlled manner. By restricting a duration of the stimulation, sensitivity of the detection and/or treatment may be improved. In some example embodiments, the effectiveness and controllability of odor stimulation for treatment of apnea is additionally improved based on dynamically changing the odorants used for stimulation. Furthermore, unwanted disturbances in sleep may be avoided based on quick dissipation of the odor stimulus. According to some example embodiments, parameters of the odor stimulation for a user may be dynamically adjusted for individual users based on a machine learning process.

According to some example embodiments, the device and method is configured to reduce snoring and/or apnea events while the user is sleeping. The respiration may be manipulated during sleep without inducing arousal.

According to some example embodiments, the device and method is additionally configured to monitor and report a plurality of different types of breathing patterns including apnea related breathing patterns and tachypnea related breathing patterns and to selectively dispense odors based on detecting an apnea related breathing pattern.

According to some example embodiments, the device and method may be operated to perform an interactive olfactory function test to identify impaired olfactory function that may be related to COVID-19 virus.

According to an aspect of some example embodiments, there is provided a method to manipulate respiration with odor dispensing, the method comprising: dispensing a dose of odor toward a user from a distance of 10 cm - 3 m from the user; and controllably dissipating the odor in the vicinity of the dispensing at pre-defined period after the dispensing.

Optionally, the dispensing together with the controlled dissipating is configured to reduce snoring and/or apnea events while the user is sleeping.

Optionally, the odor is controllably dissipated based on suspending the dose of odor on weighted granules configured to settle due to gravity at the pre-defined period, wherein at least one of size and weight of the weighted granules is selected to actuate the dissipating at the pre-defined period.

Optionally, the granules are sized with a diameter of 2 mm - 8 mm.

Optionally, the granules are selected to weigh 0.01 gm - 0.5 gm.

Optionally, the granules are silicon.

Optionally, the granules are spherical.

Optionally, the odor is a dry odor.

Optionally, the odor is controllably dissipated based on dispensing a burst of clean air at the pre-defined time period.

Optionally, the burst of clean is dispensed in a same direction as the odor.

Optionally, the odor is dispensed in coordination with a respiratory event.

Optionally, the odor is dispensed in response to output from a sensor configured to detect the respiratory event.

Optionally, the odor is dispensed at defined intervals.

Optionally, the method includes selectively dispensing one of a plurality of odors.

Optionally, the odor is selected based on a machine learning process and wherein the machine learning process is based on outputs from sensors.

Optionally, the method includes tracking position of a user and directing the dispensing toward the position, wherein the tracking is automated.

Optionally, the tracking position is based on output from a camera.

Optionally, the tracking position is based sensing breathing with one or more of sound and heat.

Optionally, the directing the dispensing is based on one or more motors configured to orient a nozzle through which the odor is dispensed.

According to an aspect of some example embodiments, there is provided a device to manipulate respiration with odor dispensing comprising: at least one cartridge including an odor in dry format; a valve configured to selectively release the odor out of the at least one cartridge; a nozzle configured to dispense discrete doses of the odor from the at least one cartridge toward a user from a distance of 10 cm - 3 m from the user; an actuator configured to actuate the dispensing through the nozzle; a controller configured to control the actuator; and a processor configured to provide input to the controller based on sensed breathing parameters.

Optionally, the controller is configured to actuate the dispensing based on output from a sensor sensing a breathing related parameter.

The sensor is a radar sensor.

Optionally, the sensor is housed in the device.

Optionally, the device includes a wireless communication protocol configured to receive data from the sensor, wherein the sensor is remote from the device.

Optionally, the device includes an array of cartridges, each including an odor in dry format; and an array of valves, each valve in the array is dedicated to controlling flow out of a cartridge in the array of cartridge, wherein the controller is configured to dynamically select an odor from the array of cartridges.

The device includes a clean air cartridge configured to dispense clean air, the clean air is configured to dissipate an odor that was previously dispensed.

Optionally, the controller is configured to actuate dispensing the clean air from the clean air cartridge at a pre-defined period after dispensing the odor from the at least one cartridge.

Optionally, the odor in the at least one cartridge is granules on which an odorant is suspended.

Optionally, the granules are formed from silicon.

Optionally, the granules have a diameter of 2 mm - 8 mm.

Optionally, the granules weigh 0.01 gm - 0.5 gm.

Optionally, the input provided by the processor is configured to actuate the dispensing for reducing snoring and/or an apnea event.

According to an aspect of some example embodiments, there is provided an odorant to manipulate respiration comprising granules including a dry odor suspended thereon, wherein the granules have a diameter of 2 mm - 8 mm and weigh 0.01 gm - 0.5 gm.

Optionally, the granules are formed from silicon.

Optionally, the granules are spherical.

According to an aspect of some example embodiments, there is provided a method to perform an olfactory function test, the method comprising: dispensing a dose of odor toward a user; requesting feedback from the user regarding the dose dispensed; evaluating olfactory function of the user based on the feedback; and reporting the evaluation.

Optionally, the method includes evaluating symptoms for COVID-19 based on the evaluating.

Optionally, the method includes dispensing a plurality of different odors toward a user in sequence and requesting feedback after each of the plurality of different odors is dispensed.

Optionally, the method includes controllably dissipating the odor in the vicinity of the dispensing at pre-defined period after the dispensing.

Optionally, the parameters of the dose of odor is pre-defined.

Optionally, the parameters of the dose of odor is dynamically actuated by a remote operator by wireless communication.

Optionally, the dose of odor is dispensed with a device as described herein above.

Optionally, the dose of odor is with an as described herein above.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWING(S)

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a simplified block diagram of an example device to detect and/or treat a respiratory conditions with odor stimulation in accordance with some example embodiments;
FIG. 2 is a simplified flow chart of an example method detect and/or treat a respiratory conditions with odor stimulation in accordance with some example embodiments;
FIG. 3 is a simplified schematic diagram showing example input and output to a machine learning engine in accordance with some example embodiments;
FIG. 4 is an example contact free device to detect and/or treat a respiratory conditions with odor stimulation in accordance with some example embodiments;
FIGS. 5A and 5B are drawings of an example nozzle for dispersing an odor shown in two different angular configurations, both in accordance with some example embodiments;
FIG. 5C is a simplified block diagram of an example motorized nozzle in accordance with some example embodiments;
FIGS. 6A and 6B showing perspective views of an example odorant compartment of another device to manipulate respiration with odor dispensing, both in accordance with some example embodiments;
FIG. 7 is a cross sectional view showing an interior construction of the example device in accordance with some example embodiments;
FIG. 8 is a blow up view of the example device all in accordance with some example embodiments;
FIG. 9 is simplified block diagram of the example device shown in FIGS. 6A-8 in accordance with some example embodiments;
FIGS. 10A, 10B and 10C are schematic drawings of an example odorant compartment (FIG. 10A) and an external casing of the odorant compartment shown in two different configurations, all in accordance with some example embodiments; and
FIG. 11 is a simplified flow chart of an example method to perform an interactive olfactory function test with the example device in accordance with some example embodiments.

### DESCRIPTION OF SPECIFIC EMBODIMENTS OF THE INVENTION

The present invention, in some embodiments thereof, relates to odor stimulation for detecting and treating a respiratory conditions and, more particularly, but not exclusively, treating apnea based on selective dispensing of odors.

Before explaining at least one embodiment of the invention in detail, it is to be understood that the invention is not necessarily limited in its application to the details of construction and the arrangement of the components and/or methods set forth in the following description and/or illustrated in the drawings. The invention is capable of other embodiments or of being practiced or carried out in various ways.

According to some example embodiments, there is provided an odor dispenser configured to dispense one or more odors as well as to controllably affect its dissipation, e.g. its removal from the vicinity of the user's nose after a defined duration. The odor dispenser may dispense doses of odor at defined intervals and then dissipate the odor after a defined duration based on input received from one or more sensors. The odor dispensed as well as the defined duration may be adjusted over time based on a machine learning process. The present inventors have found that providing burst (and/or spike) sensations by restricting a duration at which a user is able to smell the odor may provide a better sniffing reaction over repetitive cycles of odor dispensing as compared to known methods.

Dissipation is affected based on spraying a burst of clean air at a pre-defined time period after the burst of odor is dispensed. Alternatively or additionally, the odor, e.g. the molecules providing the odor is suspended on weighted particles (powder and/or granules) and dissipation is effected based on the particles settling down due to gravity and away from the user's nose. The rate at which the particles settle may be controlled based on selecting particles with a defined size, shape and/or weight.

Optionally, controllable dissipation is based on both suspending an odor on weighted particles that is sprayed and subsequently spraying a burst of clean air to direct the particles away from the user's nose. Optionally, the odors are in solid form. Optionally, the odors are stored in hermetically sealed capsule or compartment.

According to some example embodiments, the odor dispenser is a contactless device. Contactless device as used herein means a device that is operated without any physical contact with the user. In some example embodiments, the device is a standalone device that receives input based on wireless communication from a device that senses physiological parameters of a user. In other example embodiments, the device may be integral with a device that provides the sensing.

The device includes a nozzle that may be directed toward a user. The nozzle may be positioned in a desired orientation based on manual manipulation or automatically without human intervention. Optionally, a camera with processor is configured to detect a user's face and the device is configured to orient the nozzle based on input from the camera. Optionally, a tag, e.g. an electronic tag may be worn by the user or positioned near the user and the device may detect location of the tag and orient the nozzle based on the detected location. In some example embodiments, a sensor is configured to detect breathing and the device is configured to orient the nozzle based on the location at which breathing is detected. Optionally, breathing may be detected based on sensing sound or heat signature or pattern.

According to some example embodiments, the device includes a plurality of compartments configured for storing different odors and an actuator that is configured to concurrently and/or consecutively dispense more than one odor. Optionally, the odors are selected over a learning and/or calibration process.

According to some example embodiments, the device and method is configured for treating apnea based on providing an odor stimulation to alter a respiratory pattern during sleep. Optionally, the treatment is configured to avoid inducing arousal or wake-up. Optionally, the device and method is configured for altering a respiratory pattern over one or more breaths during sleep without inducing arousal or wake-up. In some example embodiments, the device and method is configured for providing sleep relaxation, respiration coaching, stress relief, posttraumatic stress disorder relief and/or deeper, more relaxed sleep. In some example embodiments, the device and method is configured to provide odor stimulation to treat coma patients. According to some example embodiments, the device is configured to detect defined breathing patterns and transmit data related to the defined breathing patterns to a remote device for reporting, storage and/or further processing. Optionally, the defined breathing patterns include apnea related breathing patterns and tachypnea related breathing patterns. Optionally, the transmission may be provided to alert a heath professional at a remote site.

According to some example embodiments, the device and method provides detecting olfactory functioning of a user. During the olfactory function test, a user may be requested to identify one or more odors selectively dispensed by the device. Optionally, the user input may be provided by via wireless communication, e.g. with a smart phone. Based on input from the user, the device and method may provide an evaluation of the user's current olfactory function.

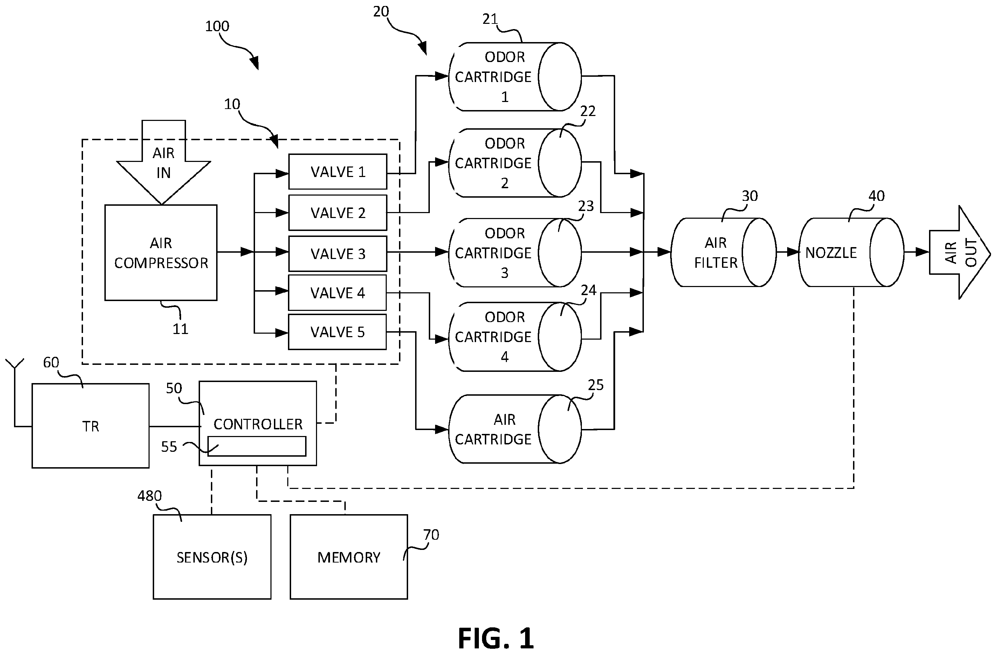

Reference is now made to FIG. 1 showing a simplified block diagram of an example device to detect and/or treat a respiratory conditions with odor stimulation in accordance with some example embodiments. According to some example embodiments, a device 100 includes an array of cartridges 20 with selected odors, e.g. cartridges 21, 22, 23 and 24 each including a different odor. It is noted that five cartridges in array 20 is shown as an example. Optionally, device 100 may have one cartridge, less than five cartridges or more than five cartridges. Optionally device 100 (and array 20) additionally includes a cartridge 25 without an odor and instead with a clean air filter. Optionally, the clean air filter is a free flow cartridge through which air that has not been odorized may be dispensed. Optionally, an array of valves 10 includes a dedicated valve for each cartridge in array 20 that selectively controls flow through the cartridges. An air compressor 11 of device 100 actuates the odor or clean air dispensing and may direct air through one or more cartridges 21, 22, 23, 24 and 25 in array 20 via one or more valves in array 10 and through a nozzle 40. Optionally, prior to dispensing, the odor or air with nozzle 40, the air may be filtered with air filter 30. Air filter 30 may filter clumps or particles above a pre-defined size for safety purposes, e.g. to prevent a user from inhaling larger particles or clumps.

A controller 50 included in device 100 may control operation of device 100. In some example embodiments, controller 50 controls operation of the air compressor and array of valves 10 based on which timing for dispensing one or more odors in array 20 as well as a dose and/or rate at which the odor is dispensed may be dynamically controlled by device 100. In some example embodiments, controller 50 is also configured to dynamically control an orientation of nozzle 40 so that the odor or air dispensed may be directed at a selected direction. For example, device 100 may include a motor that is configured to move nozzle 40 and controller 50 may control operation of the motor. Optionally, nozzle 40 includes a valve that is controlled with controller 50. A valve of nozzle 40 may be a safety valve or a valve based on which a rate of flow from air compressor 11, through one or more cartridges in array 20 and through nozzle 40 may be controlled. Optionally a valve of nozzle 40 may prevent leakage of the odors from the cartridges into the room.

According to some example embodiments, controller 50 receives input from one or more sensors 480 and controls operation of device 100 based on the input received. According to some example embodiments, at least one of sensors 480 is configured to sense breathing patterns of a user. Optionally, at least one of sensors 480 is configured to sense sniffing in response to an odor stimulation. Optionally, sensors 480 additionally includes sensors that monitor a plurality of physiological parameters. Optionally, some or all of sensors 480 are contactless and/or remote sensors that sense parameters related to a user without physically contacting the user. Sensors 480 include a radar sensor, and may include a temperature sensor, and/or an acoustic sensor. In some example embodiments, sensors 480 include one or more sensor physically on the user. In some example, one of sensors 480 is configured to sense brain activity, EEG sensors. Optionally, at least one of sensors 480 may sense change in brain activity based on the odor stimulation by the device.

According to some example embodiments, controller 50 is configured to release a dose of odor from one or more cartridges in array 20 at defined intervals and/or for a defined duration. The cartridges selected, the defined intervals and the defined duration may be based on output from sensor(s) 480 and/or from data stored in memory 70. Memory 70 may be memory included in device 100 or may be cloud memory that is accessible to controller 50 based on a wired or a wireless communication protocol. Controller 50 includes or is associated with processing capability. Optionally, controller 50 may receive input from sensors 480 and detect and/or predict an apnea event based on the input received. In some example embodiments, sensors 480 may also detect other respiratory conditions. Optionally, tachypnea may be detected with sensors 480. Optionally, one or more parameters of the odor stimulation provided by device 100 is defined based on the detection and/or the predication. Optionally, sniffing may be detected and used to monitor loss of smell symptoms associated with a COVID-19 patient. Optionally, controller 50 includes or is associated with a machine learning engine 55 configured to adjust parameters of the odor stimulation provided by device 100 based on data accumulated over time by device 100. According to some example embodiments, controller 50 may transmit data accumulated over time with a transceiver 60 to a remote site, e.g. by wireless transmission. The data accumulated may be related to apnea, may also be related to other respiratory conditions, e.g. tachypnea, may be related to other physiological conditions and may be related to physiological condition. Optionally, data accumulated and/or sensor output may be transmitted to a remote medical service center for monitoring a user, e.g. a COVID-19 patient remotely. Optionally, COVID-19 patients may be monitored remotely with device 100. Optionally, breathing patterns associated with COVID-19 may be detected with device 100 while a user is sleeping and may be reported to the user and/or medical personal. In some example embodiments, device 100 may also receive input from a remote site with transceiver 60. Input may be received for example from a remote medical service center. Optionally, output from sensors 480 may be monitored by a medical personnel remotely based on communication via transceiver 60. Optionally, operation of device 100 may be controlled by a medical personnel remotely based on communication via transceiver 60.

Optionally, data accumulated and/or sensor output may be transmitted to a remote medical service center for monitoring a patient in a coma or monitoring a user suffering from posttraumatic stress disorder. Optionally, data accumulated and/or sensor output may be transmitted to a remote medical service center for monitoring treatment of a patient with odor stimulation, e.g. apnea patient, coma patient or posttraumatic stress disorder patient.

Controller 50 is configured to actuate dispensing a burst of clean air via air cartridge 25 after releasing a burst of odor from one or more of cartridges 21, 22, 23 and 24. The clean air is configured to dissipate the odor that has been released or at least move the odor away from a vicinity of user's nose. In this manner a duration over which a user experiences the odor stimulation may be controllably limited with device 100.

According to some example embodiments, one or more cartridges in cartridge array 20 include solid particles and/or granules on which an odor is suspended. Optionally, the particles are silicon balls or flakes having a defined geometry and weight. In some example embodiments, the particles have a diameter of 2 mm - 8 mm and weigh between 0.01 gm - 0.5 gm. According to some example embodiments, the solid particles are configured to be dispersed in the air and then to settle to the ground based on its weight. The settling to the ground may provide for diverting the odor away from the user so that the user does not smell the odor. In this manner the duration over which a user receives an odor stimulation may be limited.

Optionally, duration of the odor stimulation is controlled based on both using odorized particles, e.g. silicon balls and dispensing a burst of clean air after dispensing a dose of the odorized particles. The burst of clean air may provide for displacing the particles that may otherwise settle on the user's bed or may further displace the particles. Alternatively, duration of the odor stimulation is controlled based on one of these methods as opposed to both. According to some example embodiments, parameters of the odor stimulation is selected based on collected data as well as based on a machine learning process adapted to learn what stimulation provides the best results for the user. In some example embodiments, the parameters are selected to avoid episodes of apnea based on initiating a sniffing reaction at a desired time in a monitored breathing pattern of a user without arousing the user.

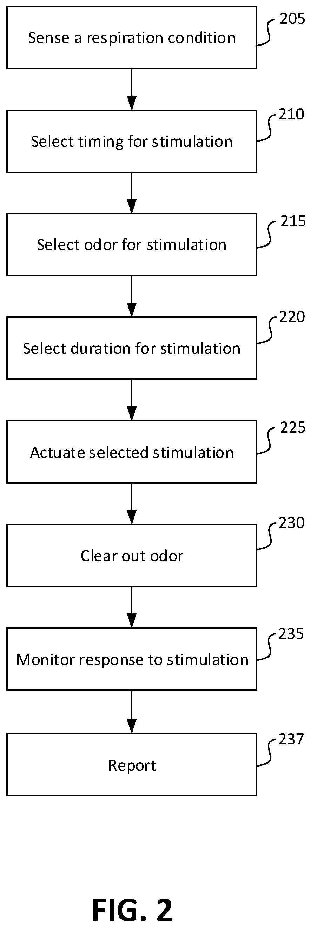

FIG. 2 is a simplified flow chart of an example method detect and/or treat a respiratory conditions with odor stimulation in accordance with some example embodiments. According to some example embodiments, a respiration condition is monitored or predicted with one or more sensors (block 205) and based on output from the sensors, parameters for odor stimulation may be determined. Selected parameters for odor stimulation may include one or more of timing for stimulation (block 210), type of odor for stimulation (block 215) and duration for stimulation (block 220). Based on the selected parameters, an odor stimulation may be actuated (block 225). The odor stimulation may be repeated at defined intervals or may be single odor stimulation based on sensed parameters. According to some embodiments, odor dispensing is followed by active dissipation of the odor (block 230). The dissipation is configured to clear the odor from a vicinity of the user. In this manner, the stimulation to the user may be provided in a concentrated burst. In some example embodiments, the response to the odor stimulation is monitored (block 235). Adjustments in one or more parameters of subsequent odor stimulations may be made based on the monitoring. Optionally, a report may be generated and transmitted by wireless transmission to a user and/or medical personnel (block 237). In some example embodiments, the report may summarize for example a quality of sleep, the number of apnea events sensed, detection of other respiratory conditions such as tachypnea and the odor stimulations provided. Optionally, the report may summarize a respiratory condition of a COVID-19 patient.

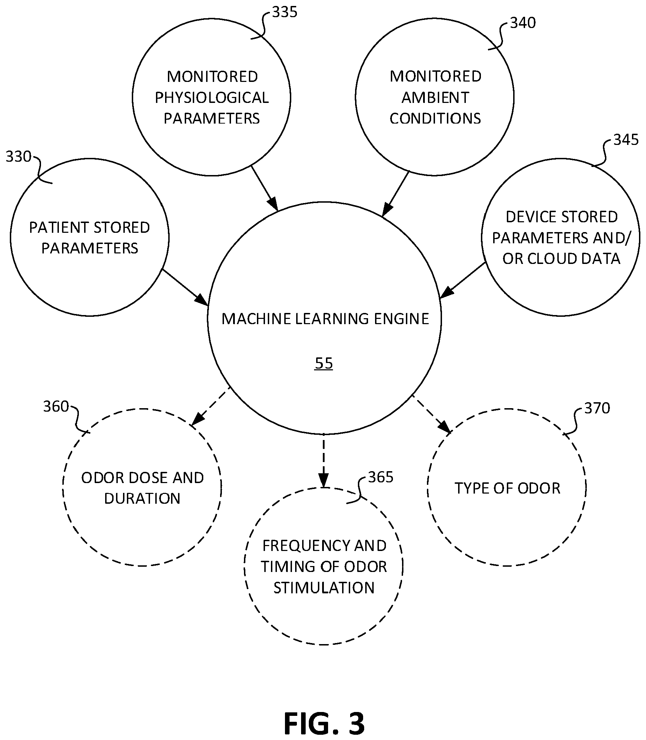

FIG. 3 is a simplified schematic diagram showing example input and output to a machine learning engine in accordance with some example embodiments. According to some example embodiments, device 100 includes a machine learning engine 55 that is configured to receive data over time and based on the data received, machine learning engine 55 may learn what parameter values for odor stimulation will provide a desired change in a respiration pattern of a user. Optionally, device 100 including machine learning engine 55 is configured to reduce episodes of apnea for a user while the user is sleeping and without arousing the user with the odor stimulation. According to some example embodiments, parameter values that may be adjusted include odor dose and/or duration (360), frequency and/or timing of odor stimulation (365) and type of odor. One or more of these parameters may be user sensitive. According to some example embodiments, a desired change in a respiration pattern may be achieved based on defining dedicated parameters values for a specific user, at a specific time and in specific ambient conditions.

According to some embodiments, the machine learning engine adjusts the parameter values based on input from one or more sensors monitoring physiological parameters of a user (335) and/or ambient conditions (340). Physiological parameters may include breathing pattern, movement of the user, sleep phase and snoring. The physiological parameters may be sensed with sensor(s) 480 (FIG. 1). Ambient conditions may include temperature in the room, humidity in the room and lighting conditions. Machine learning engine 55 may also receive user stored parameters (330) such as age, gender, weight and medical condition of the user. Optionally, machine learning engine may also receive data from a remote device and/or from cloud memory (345). Data received obtained over a calibration procedure or based on data collected from other users. Data provided to machine learning engine 55 may be used to learn the type of odor stimulation that provides a desired change in a respiration pattern, e.g., that provides a desired sniffing reaction at a defined time that may avoid an apnea episode.

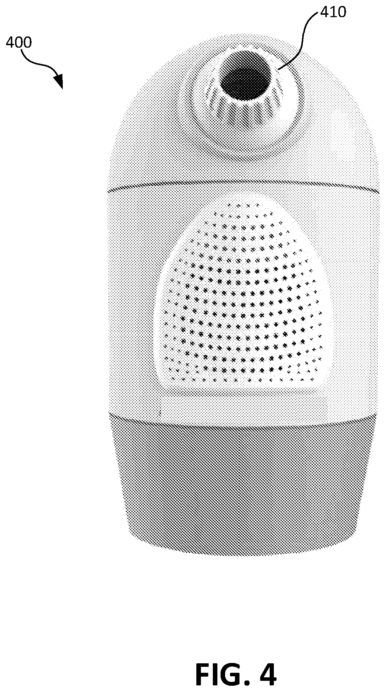

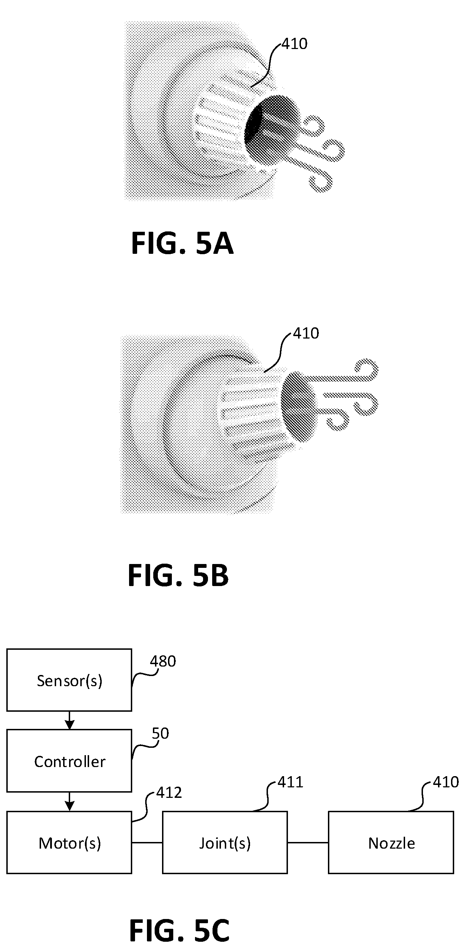

FIG. 4 is an example contact free device to detect and/or treat a respiratory conditions with odor stimulation and to FIGS. 5A and 5B are drawings of an example nozzle for dispersing an odor shown in two different angular configurations, both in accordance with some example embodiments. Device 400 may be a standalone device that is suitable for positioning on a dresser, night table, hung on a wall or placed on the floor in a user's bedroom. Device 400 may be positioned at a distance of between 10 cm - 3 m from a user. Device 400 may include a nozzle 410 through which the odor stimulation is dispensed and also through which a burst of clean air may be dispensed to dissipate the odor stimulation. In some example embodiments, nozzle 410 may be integrated with a shoulder joint so that an orientation of nozzle 410 may be adjusted manually.

Reference is now made to FIG. 5C showing a simplified block diagram of an example motorized nozzle in accordance with some example embodiments. Optionally, device 100 includes a motor 412, more than one motor or other actuator and control capability, e.g. with controller 50 to adjust orientation of nozzle 410 without human intervention. Optionally, nozzle 410 may be directed toward a user or a user's nose. Optionally, orientation of nozzle 410 may be dynamically adjusted as the user moves during sleep so that the odor stimulation is dispensed toward the user or the user's nose. In some example embodiments, controller 50 selects orientation of nozzle 410 based on outputs from one or more sensors 480. In some example embodiments, one or more sensors 480 includes at least one sensor configured to detect a breathing and device 400 is configured to orient the nozzle based on the location at which breathing is detected. Optionally, breathing may be detected based on sensing one or more of sound, heat signature or pattern and/or chest movement. Controller 50 may then actuate movement of nozzle 410 mounted or connected to one or more mechanical joints 411 to the desired orientation using one or more motors 412.

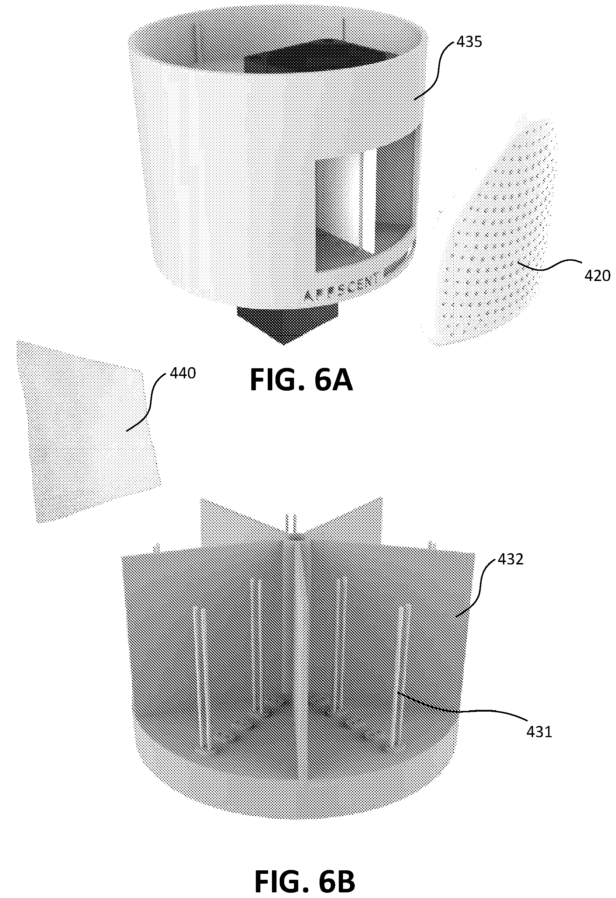

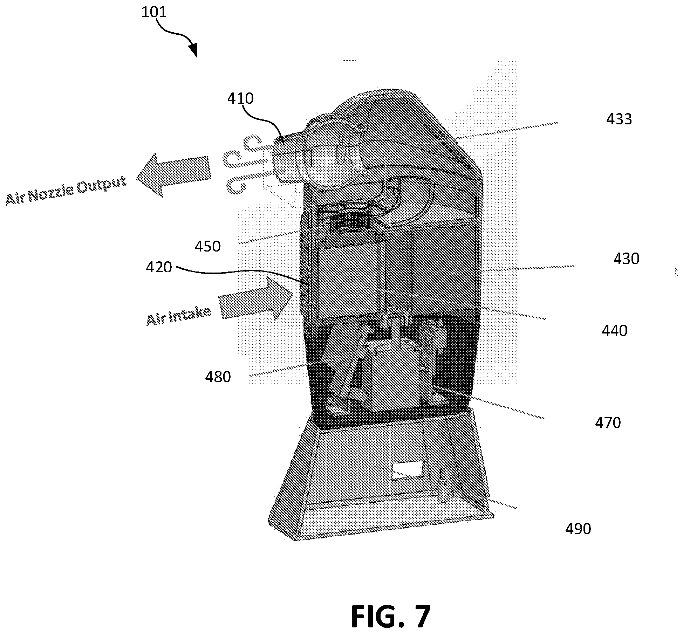

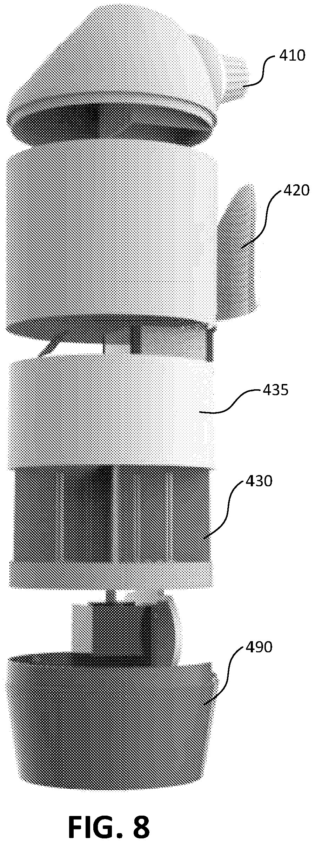

Reference is now made to FIGS. 6A and 6B showing perspective views of an example odorant compartment of another device to manipulate respiration with odor dispensing, FIG. 7 showing a cross sectional view showing an interior construction of the example device and to FIG. 8 showing a blow up view of the example device all in accordance with some example embodiments. According to some example embodiments, a device 101 (FIG. 7) is configured to house one or more pouches 440 including an odorized powder in a carousel 430 that is covered with cover 435. The carousel may have struts for supporting pouches 440 and partitioning walls 432 to separate the odor from each of pouches 440. Air intake may be via vent 420. An odor may be selected by rotating carousel 430 and aligning a selected odor with vent 420. A fan blower 450 may suction air through vent 420 and the selected compartment and thereby actuate dispensing a burst of odor stimulation via nozzle 410. Optionally, device 101 includes a circulation chamber 433 for mixing the odor prior to dispensing. Optionally, a motor 470 is configured to rotate carousel 440 and align a compartment with vent 420. Optionally, motor 470 may rotate carousel 440 while dispensing to mix two or more odors.

In some example embodiments, device 101 includes, one or more sensors 480. Optionally, one or more sensors 480 includes a sensor to senses breathing patterns. The sensor(s) for sensing breathing patterns is a radar sensor. Optionally, one or more sensors 480 additional include a sensor that senses additional physiological parameters for monitoring the user. Optionally device 101 additionally includes processing capability configured to process output from one or more sensors 480. Optionally, one or more sensors 480 includes a sensor configured to monitor breathing patterns associated with COVID-19 virus. Optionally, a processor, power supply and controller may be housed in a base 490 of device 101.

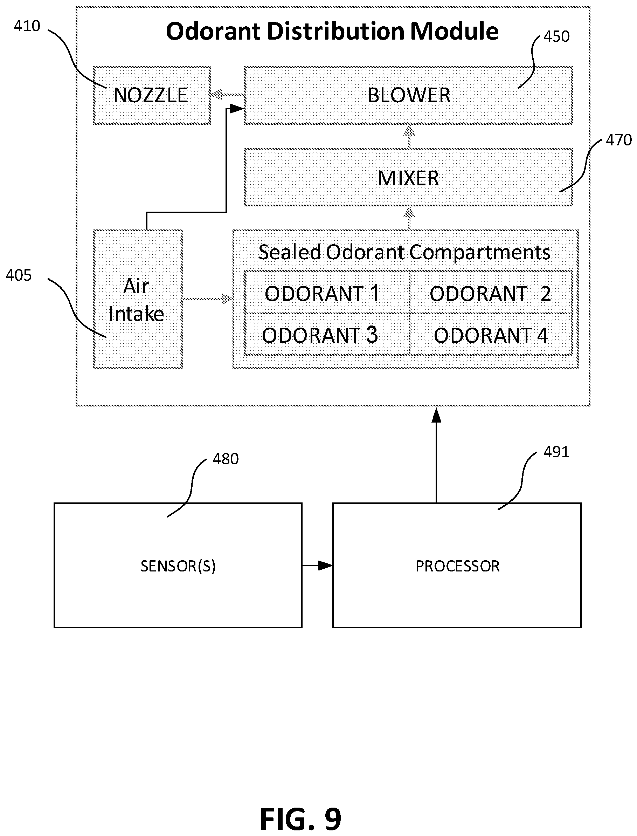

FIG. 9 is simplified block diagram of the example device shown in FIGS. 6A-8. In some example embodiments each of odorants 1, 2, 3, and 4 are example odors in a form of a powder, each of which is stored in a dedicated pouch 440 and each pouch 440 is housed in a compartment of carousel 430. The compartment may be sealed with cover 435 optionally formed with silicon. Based on input from a sensor 480, a processor 491 (with controller) defines an odor stimulation. The controller actuates movement of mixer 470 to position carousel 430 in a desired orientation and also actuates blower 450. Blower 450 suctions air through air intake 405, into the selected compartment(s); and out of nozzle 410. Optionally, mixer 470 rotates carousel during operation of blower 450 to mix more than one odor.

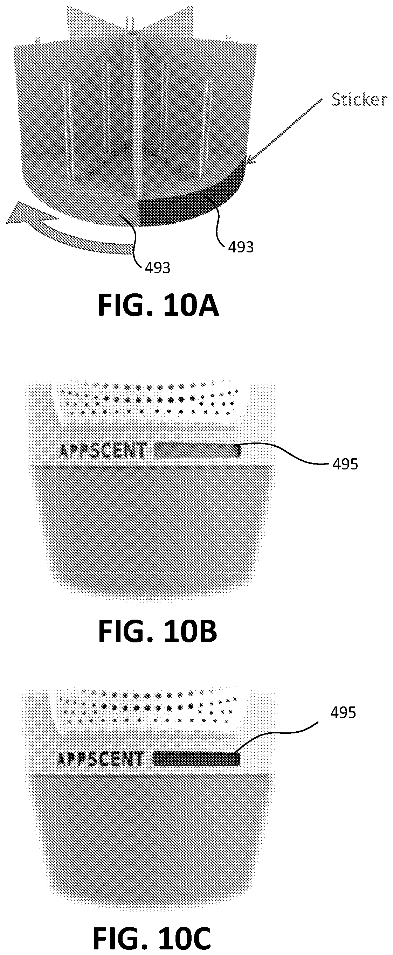

FIGS. 10A, 10B and 10C are schematic drawings of an example odorant compartment (FIG. 10A) and an external casing of the odorant compartment shown in two different configurations, all in accordance with some example embodiments. In some example embodiments, a user may manually select an odor for the odor stimulation. Optionally, the user may rotate carousel 430 based on rotating base 490. Indication of the odor selected may be visualized based on stickers 493 that show through a window 495.

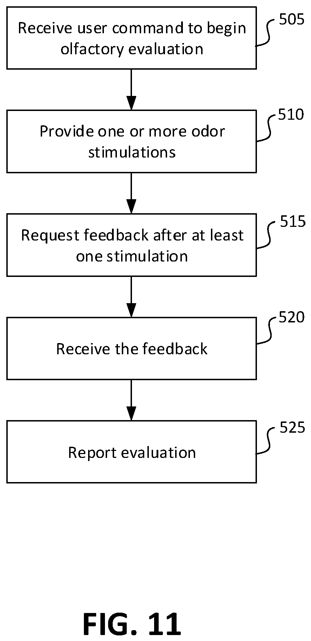

Reference is now made to FIG. 11 showing a simplified flow chart of an example method to perform an interactive olfactory function test with the example device in accordance with some example embodiments. According to some example embodiments, a device to detect and/or treat a respiratory conditions with odor stimulation as described herein may include a pre-defined olfactory evaluation mode that may be initiated based on receiving a command from a user to begin an olfactory evaluation (block 505). The command may be received by wireless communication, e.g. with a dedicated application on a smart phone. Optionally, the device includes a dedicated switch or button based on which a user may provide the command. According to some example embodiments, during the test, the device provides one or more odor stimulations (block 510). Optionally, a plurality of different odor stimulations are provided. According to some example embodiments, the odor stimulation is a burst and/or spike odor stimulation as described herein. In response to each of the odor stimulation, a user is requested to provide feedback to evaluate the odor sensed and thereby determine if the user sensed the odor stimulation and optionally to what degree, e.g. strong odor, weak odor (block 515). The input may be provided for example with the dedicated application on the smart phone. The device receives the feedback (block 520) and optionally provides a report (block 525). In some example embodiments, the device and the method as described herein may be used to determine if a person has impaired olfactory function associated with COVD-19 virus and to monitor the olfactory function. In some example embodiments, the evaluation is reported to a remote medical facility and/or personnel. In some example embodiments, a medical personal may remotely provide commands to the device to control operation of the device and the olfactory function test. Optionally, the remote medical personal may perform the test interactively with the user. The medical personal may transmit commands to the device to provide a selected odor stimulation and may ask the user for feedback based on the stimulation provided. Optionally, the feedback may be provided by telephone.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

A series of experiments were performed to test duration of an odor stimulation based on methods described herein. Rigid silicon polymer granules that store odor molecules in a completely dry form were used to provide the odor stimulation. 30 granules were dispensed over 10 seconds using compressed air. The compressed air pressure was 2 bars. Presence of the odor was monitored at 10 cm, 20 cm and 30 cm from the device dispensing the odor. Prior to dispensing a baseline level was measured. The parameters measured included number of particles (ppb) and dissipation time. A Volatile organic compounds (VOC) meter device was used for this purpose. The unit name of the device used was ppbRAE 3000(PGM-7340) and the unit firmware version used was V1.06.

### Example 1

Odor tested: rosemary
A base line level for rosemary was 1200 ppb.
Table 1 includes the results obtained after dispensing 30 granules of the rosemary odor. Odor dissipation time is defined as the time it takes to detect a base line level.

**Table 1: Rosemary Scent Test Results**

| Di stance | Number of Particles | Odor Dissipation Time |
|---|---|---|
| 10 cm | 3700 | 1.42 minutes |
| 20 cm | 4800 | 1.10 minutes |
| 30 cm | 4300 | 1.3 minutes |

After dispensing the rosemary odor, a burst of clean air was dispensed over duration of 10 seconds. The odor dissipation time was reduced by an average of 40 seconds based on the dispensing of clean air.

### Example 2

Odor tested: apple
A base line level for apple scent was 1600 ppb.
Table 2 includes the results obtained after dispensing 30 granules of the apple odor.

**Table 2: Apple Scent Test Results**

| Distance (cm) | Number of Particles (ppm) | Odor Dissipation Time |
|---|---|---|
| 10 | 2300 | 45 seconds |
| 20 | 2800 | 42 seconds |
| 30 | 2500 | 40 seconds |

### Example 3

Odor tested: Breath
A base line level for "breath" scent was 17 ppm.
Table 3 includes the results obtained after dispensing 30 granules of the breath odor.

**Table 3: Breath Scent Test Results**

| Distance (cm) | Number of Particles | Odor Dissipation Time |
|---|---|---|
| 10 | 13 ppm | 2.2 minutes |
| 20 | 10000 ppb | 2.0 minutes |
| 30 | 8000 ppb | 2.1 minutes |

### Example 4

Odor tested: Gum
A base line level for gum scent was 17 ppm.
Table 4 includes the results obtained after dispensing 30 granules of the gum odor.

**Table 4: Gum Scent Test Results**

| Distance (cm) | Number of Particles | Odor Dissipation Time |
|---|---|---|
| 10 | 13 ppm | 1.1 minutes |
| 20 | 1000 ppb | 1.1 minutes |
| 30 | 7500 ppb | 1.1 minutes |

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.
Citation or identification of any reference in this application shall not be construed as an admission that such reference is available as prior art to the present invention. To the extent that section headings are used, they should not be construed as necessarily limiting.

## Claims

1. A device (100, 101) to selectively provide an odor stimulation comprising:
a radar sensor (480) configured for contactless sensing of a breathing related parameter;
a cartridge (21, 22, 23, 24) including an odor in dry format;
a valve (10) configured to selectively release the odor out of the cartridge (21, 22, 23, 24);
a nozzle (40, 410) configured to dispense discrete doses of the odor from the cartridge (21, 22, 23, 24) toward a user from a distance of 10 cm - 3 m from the user;
an actuator (11) configured to actuate the dispensing through the nozzle (40, 410);
a clean air cartridge (25) configured to dispense clean air to ensure dissipation of previously dispensed odor away from the user;
a controller (50) configured to control the actuator (11); and
a processor (491) configured to provide input to the controller (50) based on output from said sensor (480).

2. The device (100, 101) of claim 1, wherein the processor (491) is configured to identify occurrences of an abnormal breathing pattern and wherein the controller (50) is configured to transmit a report of the abnormal breathing pattern.

3. The device (100, 101) of claim 2, wherein the processor (491) is configured to identify at least one of: (i) occurrences of tachypnea, and (ii) breathing pattern associated with COVID-19.

4. The device (100, 101) of any one of claims 1-3, comprising:
an array of cartridges (20), each including an odor in dry format; and
an array of valves (10), each valve in the array (10) is dedicated to controlling flow out of a cartridge (21, 22, 23, 24) in the array of cartridges (20), wherein the controller (50) is configured to dynamically select an odor from the array of cartridges (20).

5. The device (100, 101) of any one of claims 1-4, wherein the odor in the cartridge (21, 22, 23, 24) is granules on which an odorant is suspended.

## Patentansprüche

1. Eine Vorrichtung (100, 101) zur selektiven Bereitstellung einer Geruchsstimulation, umfassend:
einen Radarsensor (480), der zur berührungslosen Erfassung eines atmungsbezogenen Parameters ausgelegt ist;
einer Kartusche (21, 22, 23, 24), die einen Geruch in trockener Form enthält;
ein Ventil (10), das so konfiguriert ist, dass es den Geruch selektiv aus der Kartusche (21, 22, 23, 24) freisetzt;
eine Düse (40, 410), die so konfiguriert ist, dass sie diskrete Dosen des Geruchs aus der Kartusche (21, 22, 23, 24) in Richtung eines Benutzers aus einer Entfernung von 10 cm bis 3 m vom Benutzer abgibt;
einen Aktuator (11), der so konfiguriert ist, dass er die Abgabe durch die Düse (40, 410) auslöst;
eine Frischluftkartusche (25), die so ausgelegt ist, dass sie Frischluft abgibt, um die Verdrängung des zuvor abgegebenen Geruchs vom Benutzer weg sicherzustellen;
eine Steuerung (50), die so konfiguriert ist, dass sie den Aktuator (11) steuert; und
einen Prozessor (491), der so konfiguriert ist, dass er der Steuerung (50) auf der Grundlage der Ausgabe des Sensors (480) Eingaben bereitstellt.

2. Vorrichtung (100, 101) nach Anspruch 1, wobei der Prozessor (491) so konfiguriert ist, dass er das Auftreten eines abnormalen Atemmusters identifiziert, und wobei die Steuerung (50) so konfiguriert ist, dass sie einen Bericht über das abnormale Atemmuster überträgt.

3. Vorrichtung (100, 101) nach Anspruch 2, wobei der Prozessor (491) so konfiguriert ist, dass er mindestens eines der folgenden identifiziert: (i) das Auftreten von Tachypnoe und (ii) ein mit COVID-19 assoziiertes Atemmuster.

4. Die Vorrichtung (100, 101) nach einem der Ansprüche 1 bis 3, umfassend:
eine Anordnung von Kartuschen (20), von denen jede einen Geruch in trockener Form enthält; und
eine Anordnung von Ventilen (10), wobei jedes Ventil in der Anordnung (10) dazu bestimmt ist, den Ausfluss aus einer Kartusche (21, 22, 23, 24) in der Anordnung von Kartuschen (20) zu steuern, wobei die Steuerung (50) so konfiguriert ist, dass sie dynamisch einen Geruch aus der Anordnung von Kartuschen (20) auswählt.

5. Vorrichtung (100, 101) nach einem der Ansprüche 1 bis 4, wobei der Geruch in der Kartusche (21, 22, 23, 24) aus Granulat besteht, an dem ein Geruchsstoff suspendiert ist.

## Revendications

1. Dispositif (100, 101) pour fournir de manière sélective une stimulation olfactive comprenant:
un capteur radar (480) configuré pour la détection sans contact d'un paramètre lié à la respiration;
une cartouche (21, 22, 23, 24) comprenant une odeur en format sec;
une vanne (10) configurée pour libérer de manière sélective l'odeur de la cartouche (21, 22, 23, 24);
une buse (40, 410) configurée pour dispenser des doses discrètes de l'odeur à partir de la cartouche (21, 22, 23, 24) vers l'utilisateur, à une distance de 10 cm à 3 m de l'utilisateur;
un actionneur (11) configuré pour actionner la dispensation par la buse (40, 410);
une cartouche d'air pur (25) configurée pour dispenser de l'air pur afin d'assurer la dissipation de l'odeur précédemment dispensée loin de l'utilisateur;
un contrôleur (50) configuré pour contrôler l'actionneur (11); et
un processeur (491) configuré pour fournir au contrôleur (50) des données d'entrée en fonction des données de sortie dudit capteur (480).

2. Dispositif (100, 101) selon la revendication 1, dans lequel le processeur (491) est configuré pour identifier des occurrences d'un rythme respiratoire anormal et dans lequel le contrôleur (50) est configuré pour transmettre un rapport du rythme respiratoire anormal.

3. Dispositif (100, 101) selon la revendication 2, dans lequel le processeur (491) est configuré pour identifier au moins un parmi: (i) des occurrences de tachypnée, et (ii) un rythme respiratoire associé à la COVID-19.

4. Dispositif (100, 101) selon l'une quelconque des revendications 1 à 3, comprenant:
un réseau de cartouches (20), chacune comprenant une odeur en format sec; et
un réseau de vannes (10), chaque vanne dans le réseau (10) étant dédiée au contrôle du débit sortant d'une cartouche (21, 22, 23, 24) dans le réseau de cartouches (20), le contrôleur (50) étant configuré pour sélectionner dynamiquement une odeur parmi le réseau de cartouches (20).

5. Dispositif (100, 101) selon l'une quelconque des revendications 1 à 4, dans lequel l'odeur dans la cartouche (21, 22, 23, 24) se présente sous forme de granules sur lesquels un odorant est en suspension.
